# EUROPEAN PATENT APPLICATION

(11) **EP 4 793 292 A1**
(43) Date of publication of application: **19.08.2026**
(21) Application number: 24884751.9
(22) Date of filing: 29.10.2024
(51) Int. Cl.: C07K 19/00, A61K 38/17, C07K 14/00, A61K 38/00

(54) **POLYPEPTIDE DERIVATIVE, PHARMACEUTICAL COMPOSITION THEREOF AND USE THEREOF**

(30) Priority: 01.11.2023 CN 202311442146
(71) Applicant: Biocells (Beijing) Biotech Co., Ltd., Beijing 100070 (CN)
(72) Inventor: HAN, Huamin, Beijing 100070 (CN); JI, Qi, Beijing 100070 (CN); FENG, Siliang, Beijing 100070 (CN)
(74) Representative: Meissner Bolte Nürnberg
(86) International application number: PCT/CN2024/128231
(87) International publication number: WO 2025/092748

(57) **Abstract**

A polypeptide derivative or a pharmaceutically acceptable salt thereof. By means of introducing a fatty acid side chain at a specific site of an ISP polypeptide and/or forming a cyclic structure in the polypeptide by means of generating an amide bond between E and K inside the polypeptide, the stability and activity of the resulting polypeptide derivative are significantly improved. The present invention can be better used for preparing a drug for treating diseases related to nerve injuries.

## Description

This application claims priority to Chinese Patent Application No. 202311442146.7, filed on November 1, 2023, entitled "Polypeptide Derivative, Pharmaceutical Composition Thereof and Use Thereof", the content of which should be understood to be incorporated into this application by way of reference.

### Technical Field

The present application relates to the technical field of polypeptide derivatives and applications thereof, and in particular relates to a polypeptide derivative for treating a disease associated with nerve injury.

### Background

Nerve injury leads to the generation of glial scars, and the role of which is mainly to repair the blood-brain barrier (BBB), isolate the injured site from healthy tissues to prevent further injury and initiate the healing process. However, in the glial scar region, the expression of inhibitory molecules such as chondroitin sulfate proteoglycans (CSPG) is upregulated, which has a strong inhibitory effect on axonal growth, blocking the repair of the injured nerve (Nat Rev Neurosci. 2004;5:146-156). Studies have shown that CSPG exerts a nerve regeneration inhibitory effect by acting on protein tyrosine phosphatase σ (PTPσ) (Science.2009; 326(5952):592-596). PTPσ and related phosphatases contain a highly conserved intracellular wedge-shaped domain consisting of 24 amino acids, which can regulate a series of downstream signal transductions. Researchers designed an intracellular sigma peptide (ISP), which consists of a 24-peptide of the wedge-shaped domain linked to the cell-penetrating peptide tat. ISP is able to bind to recombinant human PTPσ, and can attenuate the neural inhibitory effect of CSPG. Under the action of ISP, axonal growth can break through the CSPG region, and it is dose-dependent (Nature. 2015; 518:404-40). In vivo experiments suggest that ISP can promote functional recovery in rats with spinal cord injury. After ISP dosing, bladder function, Basso-Beattie-Bresnahan (BBB) scores, and grid-walking motor tests all have significant improvements. However, ISP is a 35-peptide entirely composed of natural amino acids, with poor metabolic stability, which seriously affects its druggability.

### Summary

Based on the above background, the present application provides a polypeptide derivative or a pharmaceutically acceptable salt thereof, wherein the polypeptide derivative is derived from the following polypeptide sequence:
NH₂-GRKKRRQRRRX₁-DMAEHX₂EX₃LKAX₄DSLKLSX₅EYESI-NH₂,
wherein:
X₁ is selected from C or absent;
X₂ is selected from M or T;
X₃ is selected from R, K, A, F, L, D, E or Cit;
X₄ is selected from N or K;
X₅ is selected from Q or K;
and wherein,
when X₁ is C, the polypeptide derivative introduces a fatty acid side chain at a position of the polypeptide selected from the group consisting of: position 21, position 23 and position 27, and/or
the polypeptide derivative forms an intramolecular cyclic structure by linking side chains of E and K through an amide bond at positions of the polypeptide selected from the group consisting of: position 15 and position 21, position 18 and position 21, position 27 and position 31, position 27 and position 33, and position 30 and position 33, wherein when position 30 and position 33 are linked, X₅ at position 30 is K;
when X₁ is absent, the polypeptide derivative introduces a fatty acid side chain at a position of the polypeptide selected from the group consisting of: position 20, position 22 and position 26, and/or
the polypeptide derivative forms an intramolecular cyclic structure by linking side chains of E and K through an amide bond at positions of the polypeptide selected from the group consisting of: position 14 and position 20, position 17 and position 20, position 26 and position 30, position 26 and position 32, and position 29 and position 32, wherein when position 29 and position 32 are linked, X₅ at position 29 is K.

In another aspect, the present application further provides a pharmaceutical composition comprising the polypeptide derivative or the pharmaceutically acceptable salt thereof described herein.

In another aspect, the present application further provides the use of the polypeptide derivative or the pharmaceutically acceptable salt thereof described herein or the pharmaceutical composition described herein in the preparation of a medicament for treating a disease associated with nerve injury.

In another aspect, the present application further provides a method for treating a disease associated with nerve injury, the method comprising administering the polypeptide derivative or the pharmaceutically acceptable salt thereof described herein or the pharmaceutical composition described herein to a subject in need thereof.

In another aspect, the present application further provides the polypeptide derivative or the pharmaceutically acceptable salt thereof described herein or the pharmaceutical composition described herein, for use in treating a disease associated with nerve injury.

In the present application, on the basis of the ISP polypeptide sequence, by introducing a fatty acid side chain at a specific site, the stability of the polypeptide derivative is significantly improved, while its affinity with the receptor remains comparable or even slightly improved. The second method is to form a cyclic structure within the polypeptide by generating an amide bond between E and K inside the polypeptide to obtain a cyclic peptide derivative, and both the stability and activity of the obtained cyclic peptide derivative are significantly improved. At the same time, the two methods are further combined to both introduce a fatty acid side chain at a specific site and form a cyclic structure within the polypeptide, and the stability and activity of the obtained polypeptide derivative are also significantly improved.

Additional features and advantages of the present application will be set forth in the description which follows, and in part will become apparent from the description, or may be learned by practice of the application. Other advantages of the present application may be realized and obtained by solutions described in the description and the drawings.

### Brief Description of Drawings

The accompanying drawings are used to provide an understanding of the technical solutions of the present application, and constitute a part of the specification. They are used to explain the technical solutions of the present application together with the embodiments of the present application, and do not constitute a limitation to the technical solutions of the present application.
FIG. 1 shows the effects of various polypeptides in an embodiment of the present application on the axonal growth of human neuroblastoma (SH-SY5Y) cells in a proteoglycan environment.
FIG. 2 shows the changes in body weight over time of rats in each group after undergoing SCI surgery and being injected with physiological saline or a polypeptide. Among them, 1, 2, and 4 in the PW337 group represent the groups injected with 1 mg/kg, 2 mg/kg, and 4 mg/kg of PW337, respectively.
FIG. 3 shows the results of open field activity of rats in each group after undergoing SCI surgery and being injected with physiological saline or a polypeptide.
FIG. 4 shows the results of BBB locomotor scores of rats in each group after undergoing SCI surgery and being injected with physiological saline or a polypeptide.

### Detailed Description

Unless otherwise specified, technical and scientific terms used herein have the same meaning as commonly understood by a person skilled in the art to which this application belongs. When a certain amount, concentration, or other value or parameter is expressed in the form of a range, a preferred range, or a preferred numerical value upper limit and a preferred numerical value lower limit, it should be understood as specifically disclosing any range formed by combining any pair of range upper limit or preferred numerical value with any range lower limit or preferred numerical value, regardless of whether the range is specifically disclosed. Unless otherwise specified, numerical value ranges listed herein are intended to include the endpoints of the range and all integers and fractions (decimals) within that range.

As used herein, the terms "about" and "approximately" when used in conjunction with a numerical variable generally mean that the numerical value of the variable and all numerical values of the variable are within experimental error (e.g., within the 95% confidence interval for the mean) or within ±10% of the specified numerical value, or within a broader range.

As used herein, the expression "comprising" or similar expressions synonymous therewith such as "including", "containing" and "having", and the like are open-ended and do not exclude additional unrecited elements, steps or ingredients. The expression "consisting of" excludes any element, step or ingredient not specified. The expression "consisting essentially of" limits the scope to the specified elements, steps or ingredients, plus optionally present elements, steps or ingredients that do not materially affect the basic and novel characteristic(s) of the claimed subject matter. It should be understood that the expression "comprising" encompasses the expressions "consisting essentially of" and "consisting of".

As used herein, the expression "at least one" or "one or more" means 1, 2, 3, 4, 5, 6, 7, 8, 9 or more.

As used herein, the term "peptide" comprises a sequence of 3 or more amino acids, wherein the amino acids are naturally occurring or non-naturally occurring amino acids. Non-naturally occurring amino acids refer to amino acids that do not naturally occur in vivo, but which can be incorporated into the peptide structures described herein. Three-letter or single-letter abbreviations for amino acids are used herein to represent different amino acids, which are well known in the art.

Abbreviations for genetically encoded amino acids are conventional and are as follows: alanine (Ala or A), arginine (Arg or R), asparagine (Asn or N), aspartic acid (Asp or D), cysteine (Cys or C), glutamic acid (Glu or E), glutamine (Gln or Q), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V), citrulline (or N5-aminocarbonylornithine) (Cit).

When a three-letter abbreviation is used, unless specifically preceded by "L" or "D", or it is clear from the context in which the abbreviation is used, the amino acid may be of the L-configuration or D-configuration with respect to the α-carbon (Cα). For example, "Ala" represents alanine without designating the configuration with respect to the α-carbon, whereas "D-Ala" and "L-Ala" represent D-alanine and L-alanine, respectively.

When a single-letter abbreviation is used, a capital letter represents an amino acid of the L-configuration with respect to the α-carbon, and a lowercase letter represents an amino acid of the D-configuration with respect to the α-carbon. For example, "A" represents L-alanine and "a" represents D-alanine.

When a polypeptide sequence is presented as a string of single-letter or three-letter abbreviations (or a mixture thereof), the sequence is presented in the amino (N) to carboxyl (C) direction in accordance with conventional practice.

As used herein, the term "derivative" can refer to any compound having the same or similar core structure as a compound but having at least one structural difference (including substitution, deletion, and/or addition of one or more atoms or functional groups). The term "derivative" in relation to a peptide refers to a chemically modified (e.g., covalently modified, etc.) peptide or an analogue thereof. Typical modifications include amides, carbohydrates, alkyls, acyls, esters, and the like. The term "pharmaceutically acceptable salt" refers to a salt of a polypeptide or protein that retains the biological activity of the parent.

As used herein, numerical superscripts in a polypeptide sequence are for the purpose of better referring to the position where an amino acid is located in the polypeptide and are the numbering of the amino acids from left to right. For example, "K²¹" represents the amino acid "K" at position 21 starting from left to right. Whereas "K²⁷-E³¹" represents that a connection is formed by generating an amide bond between the amino acid "K" at position 27 and the amino acid "E" at position 31, thereby forming an intramolecular cyclic structure in the polypeptide. "N²³ mutated to K²³" represents that the amino acid "N" at position 23 has been mutated to the amino acid "K" at position 23. "After N²³ being mutated to K²³", i.e., "K²³", such an expression is intended to enable a person skilled in the art to more clearly understand the changes in the polypeptide sequence and find the position of the amino acid.

As used herein, "ring ()" in the polypeptide sequence represents that a cyclic structure is formed by the amino acids in the parentheses together within the polypeptide. For example, "ring (KLSQE)" represents that, within the polypeptide, cyclization is achieved by the connection of an amide bond between K and E, and an intramolecular ring of the polypeptide is formed by these amino acids KLSQE.

As used herein, "K()" in the polypeptide sequence represents the introduction of a fatty acid side chain on the K side chain of the polypeptide. Within the parentheses is the fatty acid side chain. In the present application, when representing a fatty acid chain, an italicized and bold manner is adopted for representation, so as to distinguish it from the amino acid sequence represented by single letters. For example, "K(***-AEEA-AEEA-γE-ODDA***)" represents the introduction of the fatty acid side chain ***-AEEA-AEEA-γE-ODDA*** at the amino acid K in the polypeptide chain.

As used herein, the Chinese explanations for abbreviations or English full names used in the present application are shown in Table 1 below:

**Table 1**

| Abbreviations or English | Meaning |
|---|---|
| Boc | tert-Butoxycarbonyl |
| DCM | Dichloromethane |
| DIEA | Diisopropylethylamine |
| DMF | N,N-Dimethylformamide |
| MeOH | Methanol |
| ACN | Acetonitrile |
| DIC | Diisopropylcarbodiimide |
| HOBt | Benzotriazole |
| Fmoc | Fluorenylmethyloxycarbonyl |
| HBTU | O-Benzotriazole-tetramethyluronium hexafluorophosphate |
| PYBOP | Benzotriazol-1-yl-oxytripyrrolidinophosphonium hexafluorophosphate |
| OtBu | -O-C(CH₃)₃ |
| Dde | 1-(4,4-Dimethyl-2,6-dioxocyclohexylidene)ethyl |
| Alloc | Allyloxycarbonyl |
| Oall | Allyl ester |
| Resin | Resins |
| Trt | Trityl |
| TFA | Trifluoroacetic acid |
| TIS | Triisopropylsilane |
| AEEA | Aminoethoxyethoxyacetic acid |
| ODDA | Octadecanedioic acid |
| C16 | Palmitoyl |
| C18 | Stearoyl |
| C14 | Myristoyl |
| C12 | Lauroyl |
| γE | γ-glutamic acid, meaning that glutamic acid participates in the generation of an amide bond with its γ-carboxyl group in the peptide sequence |
| SCI | Spinal cord injury |
| sham | Sham operation group |
| PW6, PW92, PW93, PW98, PW99, PW101, PW106, PW170, PW175, PW182, PW239, PW240, PW242, PW247, PW252, PW253, PW257, PW259, PW261, etc. | equivalent to PW-6, PW-92, PW-93, PW-98, PW-99, PW-101, PW-106, PW-170, PW-175, PW-182, PW-239, PW-240, PW-242, PW-247, PW-252, PW-253, PW-257, PW-259, PW-261, respectively; the hyphen "-" in the middle of the compound numbering can be omitted. |

General description: the hyphen "-" in the middle of the compound numbering can be omitted, and the hyphen is omitted in the numbering in some experiments, for example, PW-337 is equivalent to PW337; unless otherwise specified, the polypeptide is by default in the form of a trifluoroacetate salt.

The nerve repair mechanism of ISP is mediated by a number of endogenous repair mechanisms, including regeneration, plasticity, remyelination, immunomodulation, and synapse formation, and these repair mechanisms help to treat nerve injuries associated with nerve injury (spinal cord injury, peripheral nerve injury, traumatic brain injury, and stroke) and neurodegenerative diseases (multiple sclerosis, Alzheimer's disease, amyotrophic lateral sclerosis (ALS), frontotemporal dementia (FTD), and Parkinson's disease), having very broad potential medicinal value. However, ISP is a 35-peptide entirely composed of natural amino acids, with poor metabolic stability, which seriously affects its druggability.

The sequence of ISP is as follows:

NH2-G1R2K3K4R5R6Q7R8R9R10C11-
D12M13A14E15H16M17E18R19L20K21A22N23D24S25L26K27L28S29Q30E31Y32E33S34I35-NH2

The structure of ISP is derived from the PTPσ wedge-shaped domain of mice, whereas in the human-derived PTPσ wedge-shaped domain, M at position 17 is mutated to T, thus in the polypeptide derivatives provided herein, this site can be M or T.

Introducing a long-chain fatty acid into the side chain of the polypeptide can increase plasma protein binding, improve metabolic stability, and improve druggability. However, introducing a fatty acid side chain at certain sites may have a serious impact on the affinity of the polypeptide with the receptor, therefore, suitable modification sites are very important. The present application found that by introducing a fatty acid side chain at specific sites, the stability of the polypeptide derivative is significantly improved, while the receptor affinity remains comparable or even slightly improved. In addition, cyclization also improves the stability of the polypeptide compound. When ISP interacts with the receptor, D¹²-K²¹ and L²⁶-I³⁵ respectively form an α-helical structure, wherein the E-K salt bridge plays an important role in the stability of the α-helix. Combined with the structural features of ISP, the side chains of E and K are linked through an amide bond at appropriate sites in its two α-helical structure regions. Specific positions include, but are not limited to: K²⁷-E³¹, E¹⁸-K²¹, K²⁷-E³³, E¹⁵-K²¹ and K³⁰-E³³ (Q³⁰ is mutated to K³⁰). Among them, the two α-helical structure regions can be cyclized separately or simultaneously. In the present application, by generating an amide bond between E and K to further stabilize the α-helix to obtain a cyclic peptide derivative, it was found that both the stability and activity of some polypeptide derivatives of the present application are significantly improved.

In view of this, the present application provides a polypeptide derivative or a pharmaceutically acceptable salt thereof, wherein the polypeptide derivative is derived from the following polypeptide sequence:
NH₂-GRKKRRQRRRX₁-DMAEHX₂EX₃LKAX₄DSLKLSX₅EYESI-NH₂,
wherein:
X₁ is selected from C or absent;
X₂ is selected from M or T;
X₃ is selected from R, K, A, F, L, D, E or Cit;
X₄ is selected from N or K;
X₅ is selected from Q or K;
and wherein,
when X₁ is C, the polypeptide derivative introduces a fatty acid side chain at a position of the polypeptide selected from the group consisting of: position 21, position 23 and position 27, and/or
the polypeptide derivative forms an intramolecular cyclic structure by linking side chains of E and K through an amide bond at positions of the polypeptide selected from the group consisting of: position 15 and position 21, position 18 and position 21, position 27 and position 31, position 27 and position 33, and position 30 and position 33, wherein when position 30 and position 33 are linked, X₅ at position 30 is K;
when X₁ is absent, the polypeptide derivative introduces a fatty acid side chain at a position of the polypeptide selected from the group consisting of: position 20, position 22 and position 26, and/or
the polypeptide derivative forms an intramolecular cyclic structure by linking side chains of E and K through an amide bond at positions of the polypeptide selected from the group consisting of: position 14 and position 20, position 17 and position 20, position 26 and position 30, position 26 and position 32, and position 29 and position 32, wherein when position 29 and position 32 are linked, X₅ at position 29 is K.

In some embodiments, the polypeptide derivative introduces a fatty acid side chain at a position of the polypeptide selected from the group consisting of: K²¹, K²⁷, and the side chain amino group after N²³ being mutated to K²³.

In some embodiments, the polypeptide derivative introduces a fatty acid side chain at a position of the polypeptide selected from the group consisting of: K²⁰, K²⁶, and the side chain amino group after N²² being mutated to K²².

In some embodiments, the polypeptide derivative is cyclized by linking side chains of E and K through an amide bond at positions of the polypeptide selected from the group consisting of: K²⁷-E³¹, E¹⁸-K²¹, K²⁷-E³³, E¹⁵-K²¹, and after Q³⁰ being mutated to K³⁰-E³³.

In some embodiments, the polypeptide derivative is cyclized by linking side chains of E and K through an amide bond at positions of the polypeptide selected from the group consisting of: K²⁶-E³⁰, E¹⁷-K²⁰, K²⁶-E³², E¹⁴-K²⁰, and after Q²⁹ being mutated to K²⁹-E³².

In some embodiments, the fatty acid side chain is: ***-Y1-Y2-Y3-Y4***;
wherein, ***Y1*** can be absent; or be -NH-(CH₂-CH₂-O)ₘ₁-(CH₂)ₘ₂-CO-, wherein m1 is a positive integer of 2-10, and m2 is 1 or 2; or be L/D-Glu, the α- or γ-carboxyl group of which is connected to the side chain amino group of the backbone amino acid of the polypeptide, and the amino group of which is connected to the carboxyl group of ***Y2*** or ***Y3*** or ***Y4***;
***Y2*** can be absent; or be -NH-(CH₂-CH₂-O)ₘ₃-(CH₂)ₘ₄-CO-, wherein m3 is a positive integer of 2-10, and m4 is 1 or 2;
***Y3*** can be absent; or be -NH-(CH₂-CH₂-O)ₘ₅-(CH₂)ₘ₆-CO-, wherein m5 is a positive integer of 2-10, and m6 is 1 or 2; or be L/D-Glu, the α- or γ-carboxyl group of which is connected to the side chain amino group of the backbone amino acid of the polypeptide or the amino group of ***Y1*** or the amino group of ***Y2***, and the amino group of which is connected to the carboxyl group of ***Y4***;
***Y4*** is HOOC-(CH₂)ₙ-CO- or CH₃-(CH₂)ₙ-CO, for example, stearoyl (-C18), palmitoyl (-C16), myristoyl (-C14) or lauroyl (-C12), wherein n is a positive integer of 10-20.

In some embodiments, the fatty acid side chain is selected from: ***-AEEA-AEEA-γE-ODDA***, ***-AEEA-AEEA-γE-C18***, ***-AEEA-AEEA-γE-C16***, ***-AEEA-AEEA-γE-C14***, ***-AEEA-AEEA-γE-C12***,***-AEEA-γE-ODDA***, ***-AEEA-γE-C18***, ***-AEEA-γE-C16***, ***-AEEA-γE-C14*** or ***-AEEA-γE-C12***; preferably, the fatty acid side chain is ***-AEEA-AEEA-γE-ODDA***, ***-AEEA-AEEA-γE-C16***, ***- AEEA-γE-C16*** or ***-AEEA-γE-C12***.

In some embodiments, the amino acids in the cell-penetrating peptide tat sequence GRKKRRQRRRC (SEQ ID NO:52) in the polypeptide are all L-amino acids, or are all D-amino acids, or are a mixture of D/L-amino acids; and wherein C¹¹ at the C-terminus of the cell-penetrating peptide tat is present or absent.

In some embodiments, the amino groups at both termini of the polypeptide can be optionally substituted with a carboxyl group or an amide.

In some embodiments, the polypeptides or polypeptide derivatives provided herein are as shown in Table 2 (SEQ ID NO: 1-51) below:

**Table 2**

| SEQ ID NO: | Number | Sequences | MW |
|---|---|---|---|
| 1 | PW-5 | NH₂-GRKKRRQRRR-C-DMAEHMERLKANDSLKLSQEYESI-NH₂ | 4319 |
| 2 | PW-6 | NH₂-GRKKRRQRRR-C-DMAEHTERLKANDSLKLSQEYESI-NH₂ | 4289 |
| 3 | PW-71 | NH2-GrKKRrQrRR-C-DMAEHMERLKANDSLK(-AEEA-AEEA-γE- ODDA)LSQEYESI-NH2 | 5034 |
| 4 | PW-72 | NH2-GrKKRrQrRR-C-DMAEHMERLK(-AEEA-AEEA-γE- ODDA)ANDSLKLSQEYESI-NH2 | 5034 |
| 5 | PW-73 | NH2-GrKKRrQrRR-C-DMAEHMERLKAK(-AEEA-AEEA-γE- ODDA)DSLKLSQEYESI-NH2 | 5047 |
| 6 | PW-74 | NH₂-GrKKRrQrRR-C-DMAEHMERLKANDSLcyclo(KLSQE)YESI-NH₂ | 4301 |
| 7 | PW-75 | NH₂-GrKKRrQrRR-C-DMAEHMcyclo(ERLK)ANDSLKLSQEYESI-NH₂ | 4301 |
| 8 | PW-89 | NH2-GrKKRrQrRR-C-DMAEHTERLKANDSLK(-AEEA-AEEA-γE- ODDA)LSQEYESI-NH2 | 5004 |
| 9 | PW-90 | NH2-GrKKRrQrRR-DMAEHTERLKANDSLK(-AEEA-AEEA-γE- ODDA)LSQEYESI-NH2 | 4901 |
| 10 | PW-92 | NH2-GrKKRrQrRR-C-DMAEHTERLK(-AEEA-AEEA-γE- ODDA)ANDSLKLSQEYESI-NH2 | 5004 |
| 11 | PW-93 | NH2-GrKKRrQrRR-DMAEHTERLK(-AEEA-AEEA-γE- ODDA)ANDSLKLSQEYESI-NH2 | 4901 |
| 12 | PW-95 | NH2-GrKKRrQrRR-C-DMAEHTERLKAK(-AEEA-AEEA-γE- ODDA)DSLKLSQEYESI-NH2 | 5017 |
| 13 | PW-96 | NH2-GrKKRrQrRR-DMAEHTERLKAK(-AEEA-AEEA-γE- ODDA)DSLKLSQEYESI-NH2 | 4914 |
| 14 | PW-98 | NH₂-GrKKRrQrRR-C-DMAEHTERLKANDSLcyclo(KLSQE)YESI-NH₂ | 4271 |
| 15 | PW-99 | NH₂-GrKKRrQrRR-DMAEHTERLKANDSLcyclo(KLSQE)YESI-NH₂ | 4168 |
| 16 | PW-101 | NH₂-GrKKRrQrRR-C-DMAEHTcyclo(ERLK)ANDSLKLSQEYESI-NH₂ | 4271 |
| 17 | PW-102 | NH₂-GrKKRrQrRR-DMAEHTcyclo(ERLK)ANDSLKLSQEYESI-NH₂ | 4168 |
| 18 | PW-106 | NH₂-GrKKRrQrRR-C-DMAEHTERLKANDSLcyclo(KLSQEYE)SI-NH₂ | 4271 |
| 19 | PW-107 | NH₂-GrKKRrQrRR-DMAEHTERLKANDSLcyclo(KLSQEYE)SI-NH₂ | 4168 |
| 20 | PW-109 | NH₂-GrKKRrQrRR-C-DMAcyclo(EHTERLK)ANDSLKLSQEYESI-NH₂ | 4271 |
| 21 | PW-110 | NH₂-GrKKRrQrRR-DMAcyclo(EHTERLK)ANDSLKLSQEYESI-NH₂ | 4168 |
| 22 | PW-160 | NH₂-GrKKRrQrRR-DMAEHTERLKANDSLKLScyclo(KEYE)SI-NH₂ | 4168 |
| 23 | PW-161 | NH₂-GrKKRrQrRR-C-DMAEHTERLKANDSLKLScyclo(KEYE)SI-NH₂ | 4271 |
| 24 | PW-164 | NH2-GrKKRrQrRR-DMAEHTcyclo(ERLK)ANDSLcyclo(KLSQEYE)SI- NH2 | 4150 |
| 25 | PW-165 | NH2-GrKKRrQrRR-C- DMAEHTcyclo(ERLK)ANDSLcyclo(KLSQEYE)SI-NH2 | 4253 |
| 26 | PW-170 | NH2-GRKKRRQRRR-C-DMAEHTERLKANDSLcyclo(KLSQE)YESI- NH2 | 4271 |
| 27 | PW-173 | NH2-GrKKRrQrRR-C-DMAEHTERLK(-AEEA-AEEA-γE- ODDA)ANDSLcyclo(KLSQE)YESI-NH2 | 4986 |
| 28 | PW-174 | NH2-GrKKRrQrRR-DMAEHTERLK(-AEEA-AEEA-γE- ODDA)ANDSLcyclo(KLSQE)YESI-NH2 | 4883 |
| 29 | PW-175 | NH2-GRKKRRQRRR-C-DMAEHTERLK(-AEEA-AEEA-γE- ODDA)ANDSLcyclo(KLSQE)YESI-NH2 | 4986 |
| 30 | PW-176 | NH2-GRKKRRQRRR-DMAEHTERLK(-AEEA-AEEA-γE- ODDA)ANDSLcyclo(KLSQE)YESI-NH2 | 4883 |
| 31 | PW-177 | NH2-GrKKRrQrRR-C-DMAEHTcyclo(ERLK)ANDSLK(-AEEA-AEEA- γE-ODDA)LSQEYESI-NH2 | 4986 |
| 32 | PW-178 | NH2-GrKKRrQrRR-DMAEHTcyclo(ERLK)ANDSLK(-AEEA-AEEA-γE- ODDA)LSQEYESI-NH2 | 4883 |
| 33 | PW-179 | NH2-GRKKRRQRRR-C-DMAEHTcyclo(ERLK)ANDSLK(-AEEA- AEEA-γE-ODDA)LSQEYESI-NH2 | 4986 |
| 34 | PW-180 | NH2-GRKKRRQRRR-DMAEHTcyclo(ERLK)ANDSLK(-AEEA-AEEA- γE-ODDA)LSQEYESI-NH2 | 4883 |
| 35 | PW-181 | NH2-GRKKRRQRRR-C-DMAEHTERLKANDSLK(-AEEA-AEEA-γE- ODDA)LSQEYESI-NH2 | 5004 |
| 36 | PW-182 | NH2-GRKKRRQRRR-DMAEHTERLKANDSLK(-AEEA-AEEA-γE- ODDA)LSQEYESI-NH2 | 4901 |
| 37 | PW-337 | NH2-GRKKRRQRRR-DMAEHMERLKANDSLK(-AEEA-AEEA-γE- ODDA)LSQEYESI-NH2 | 4932 |
| 38 | PW-183 | NH2-GRKKRRQRRR-C-DMAEHTERLK(-AEEA-AEEA-γE- ODDA)ANDSLKLSQEYESI-NH2 | 5004 |
| 39 | PW-184 | NH2-GRKKRRQRRR-DMAEHTERLK(-AEEA-AEEA-γE- ODDA)ANDSLKLSQEYESI-NH2 | 4901 |
| 40 | PW-185 | NH2-GRKKRRQRRR-C-DMAEHTERLKAK(-AEEA-AEEA-γE- ODDA)DSLKLSQEYESI-NH2 | 5017 |
| 41 | PW-186 | NH2-GRKKRRQRRR-DMAEHTERLKAK(-AEEA-AEEA-γE- ODDA)DSLKLSQEYESI-NH2 | 4914 |
| 42 | PW-239 | NH₂-GrKKRrQrRR-C-DMAEHTEALKANDSLcyclo(KLSQEYE)SI-NH₂ | 4185 |
| 43 | PW-240 | NH₂-GrKKRrQrRR-DMAEHTEALKANDSLcyclo(KLSQEYE)SI-NH₂ | 4082 |
| 44 | PW-241 | NH2-GrKKRrQrRR-C-DMAEHTEALK(-AEEA-AEEA-γE- ODDA)ANDSLKLSQEYESI-NH2 | 4919 |
| 45 | PW-242 | NH2-GrKKRrQrRR-DMAEHTEALK(-AEEA-AEEA-γE- ODDA)ANDSLKLSQEYESI-NH2 | 4816 |
| 46 | PW-247 | NH2-GRKKRRQRRR-C-DMAEHTEK(-AEEA-AEEA-γE- ODDA)LKANDSLKLSQEYESI-NH2 | 4977 |
| 47 | PW-252 | NH2-GrKKRrQrRR-C-DMAEHTERLK(-AEEA-AEEA-γE- C16)ANDSLKLSQEYESI-NH2 | 4945 |
| 48 | PW-253 | NH2-GrKKRrQrRR-DMAEHTERLK(-AEEA-AEEA-γE- C16)ANDSLKLSQEYESI-NH2 | 4842 |
| 49 | PW-257 | NH2-GrKKRrQrRR-DMAEHTERLK(-AEEA-γE- ODDA)ANDSLKLSQEYESI-NH2 | 4756 |
| 50 | PW-259 | NH2-GrKKRrQrRR-DMAEHTERLK(-AEEA-γE- C16)ANDSLKLSQEYESI-NH2 | 4697 |
| 51 | PW-261 | NH2-GrKKRrQrRR-DMAEHTERLK(-AEEA-γE- C12)ANDSLKLSQEYESI-NH2 | 4641 |

In another aspect, the present application further provides a pharmaceutical composition comprising the polypeptide derivative or the pharmaceutically acceptable salt thereof described herein.

In some embodiments, the pharmaceutical composition further comprises one or more pharmaceutically acceptable components selected from the following: a diluent, a buffer, an excipient, a salt, an emulsifier, an osmotic pressure regulator, a preservative, a stabilizer, and a filler.

In some embodiments, non-limiting examples of pharmaceutically acceptable excipients suitable for use in the present disclosure include a granulating agent, a binder, a lubricant, a disintegrant, a sweetener, a glidant, an anti-adherent, an antistatic agent, a surfactant, an antioxidant, a gum, a coating agent, a colorant, a flavoring agent, a plasticizer, a suspending agent, an antimicrobial agent, a plant cellulosic material, and a spheronizing agent, and any combination thereof.

In some embodiments, the pharmaceutical composition is administered through oral, intravenous, intradermal, transdermal, intrathecal, intraarterial, intraperitoneal, intranasal, intravaginal, intrarectal, intravesical, intratumoral, topical, intramuscular, subcutaneous, mucosal, inhalation, injection, infusion, or any combination thereof.

In some embodiments, the pharmaceutical composition of the present invention can be dosed orally, such as direct oral administration, incorporation into drinking water or food, or oral gavage, etc.

In some embodiments, the dosage form of the pharmaceutical composition is selected from one or more of a capsule, a tablet, a pill, a liquid, a powder, a granule, a fine granule, a film-coated agent, a pellet, a lozenge, a sublingual agent, a peptizer, a buccal preparation, a paste, a syrup, a suspension, an elixir, an emulsion, a coating agent, an ointment, a plaster, a cataplasm, a transdermal preparation, a lotion, an inhalant, an aerosol, an injection, or a suppository.

In another aspect, the present application further provides the use of the polypeptide derivative or the pharmaceutically acceptable salt thereof described herein or the pharmaceutical composition described herein in the preparation of a medicament for treating a disease associated with nerve injury.

In some embodiments, the disease associated with nerve injury is selected from one or more of spinal cord injury, peripheral nerve injury, traumatic brain injury, stroke, multiple sclerosis, Alzheimer's disease, amyotrophic lateral sclerosis, dementia, and Parkinson's disease.

In another aspect, the present application further provides a method for treating a disease associated with nerve injury, the method comprising administering the polypeptide derivative or the pharmaceutically acceptable salt thereof described herein or the pharmaceutical composition described herein to a subject in need thereof.

In some embodiments, the disease associated with nerve injury is selected from one or more of spinal cord injury, peripheral nerve injury, traumatic brain injury, stroke, multiple sclerosis, Alzheimer's disease, amyotrophic lateral sclerosis, dementia, and Parkinson's disease.

In another aspect, the present application further provides the polypeptide derivative or the pharmaceutically acceptable salt thereof described herein or the pharmaceutical composition described herein, for use in treating a disease associated with nerve injury.

In some embodiments, the disease associated with nerve injury is selected from one or more of spinal cord injury, peripheral nerve injury, traumatic brain injury, stroke, multiple sclerosis, Alzheimer's disease, amyotrophic lateral sclerosis, dementia, and Parkinson's disease.

Multiple examples have been described in the present application, but the description is exemplary rather than limiting, and it will be apparent to a person of ordinary skill in the art that there can be more examples and implementations within the scope encompassed by the examples described in the present application. Although many possible combinations of features are shown in the accompanying drawings and discussed in the specific embodiments, many other ways of combining the disclosed features are also possible. Unless specifically limited otherwise, any feature of any example can be used in combination with any other feature in any other example, or can substitute for any other feature in any other example.

The present application includes and contemplates combinations with features known to a person of ordinary skill in the art. The examples and features already disclosed in the present application can also be combined with any conventional features to form a unique inventive scheme defined by the claims. Any feature of any example can also be combined with features from other inventive schemes to form another unique inventive scheme defined by the claims. Therefore, it should be understood that any feature shown and/or discussed in the present application can be implemented individually or in any appropriate combination. Accordingly, the examples are not subject to other limitations except those made in accordance with the appended claims and their equivalent replacements. Furthermore, various modifications and changes can be made within the protection scope of the appended claims.

In addition, when describing representative examples, the specification may have presented methods and/or processes as specific sequences of steps. However, to the extent that the method or process does not rely on the specific order of the steps described herein, the method or process should not be limited to the specific order of steps. As will be understood by a person of ordinary skill in the art, other sequences of steps are also possible. Therefore, the specific order of steps set forth in the specification should not be construed as a limitation on the claims. Furthermore, claims directed to the method and/or process should not be limited to performing their steps in the order written, and a person skilled in the art can readily understand that these sequences can vary and still remain within the spirit and scope of the examples of the present application.

For the experimental methods without specifying specific conditions in the following examples, they are generally determined according to national standards. The experimental materials without specifying sources in the following examples are all commercially available raw materials. The equipment used in each step in the following examples are all conventional equipment. If there is no corresponding national standard, it is performed according to general international standards, conventional conditions, or conditions suggested by the manufacturer. Unless otherwise defined or specified, all professional and scientific terms used in the present application have the same meaning as familiar to a person skilled in the art. In addition, any methods and materials similar or equivalent to the contents described can be applied in the methods of the present application.

The relevant materials used in the examples are shown as follows:

### Reagents

| Number | Raw Material Name | Manufacturer |
|---|---|---|
| 1 | Rink Amide AM resin | Shanghai Jizhi Biochemical Technology Co., Ltd. |
| 2 | Fmoc-Ile-OH | |
| 3 | Fmoc-Asp(OtBu)-OH | |
| 4 | Fmoc-Leu-OH | |
| 5 | Fmoc-His(Trt)-OH | |
| 6 | Fmoc-Ala-OH | |
| 7 | Boc-Gly-OH | |
| 8 | Fmoc-Tyr(tBu)-OH | |
| 9 | Fmoc-Val-OH | |
| 10 | Fmoc-Glu(OtBu)-OH | |
| 11 | Fmoc-Glu-OtBu | |
| 12 | Fmoc-Glu(OAll)-OH | |
| 13 | Fmoc-Asn(Trt)-OH | |
| 14 | FmocGln(Trt)-OH | |
| 15 | Fmoc-Lys(Boc)-OH | |
| 16 | Fmoc-Lys(Dde)-OH | |
| 17 | Fmoc-Lys(Alloc)-OH | |
| 18 | Fmoc-Cys(Trt)-OH | |
| 19 | Fmoc-Met-OH | |
| 20 | Fmoc-Ser(tBu)-OH | |
| 21 | Fmoc-Arg(Pbf)-OH | |
| 22 | Fmoc-Thr(tBu)-OH | |
| 23 | Fmoc-AEEA-OH | |
| 24 | OtBu-ODDA | |
| 25 | DIC | |
| 26 | HOBt | |
| 27 | HBTU | |
| 28 | HATU | |
| 29 | PYBOP | |
| 30 | DIEA | |
| 31 | DMF | |
| 32 | DCM | |
| 33 | MeOH | |
| 34 | ACN | |
| 35 | 4-Me-Piperidine | |
| 36 | Hydrazine hydrate solution | |
| 37 | Tetrakis(triphenylphosphine)palladium | |
| 38 | Phenylsilane | |
| 39 | TFA | |
| 40 | TIS | |

The control drugs PW-5 and PW-6 used in this experiment were synthesized by our company according to conventional synthesis methods based on the sequences reported in literatures.

### Preparation Examples: Synthesis of PW series compounds

### 1. Synthesis of PW-89

0.50 g (0.10 mmol) of Rink amide resin was weighed into a reactor, 10 mL of DCM was added to swell for 10 min, suction filtered, washed 2 times with DMF, and 25% 4-methylpiperidine/DMF (volume ratio) was added to react for 30 min to remove the Fmoc group. Suction filtered, the resin was washed 4 times with DMF and 2 times with DCM, detected by ninhydrin, and the resin solution was blue; Fmoc-Ile-OH (0.40 mmol, 4 eq.) and HBTU (0.36 mmol, 3.6 eq.) were weighed, dissolved in DMF, and DIEA (0.80 mmol, 8 eq.) was added, mixed homogeneously, added to the resin, and reacted at room temperature with magnetic stirring for 1 h, suction filtered, sequentially washed 3 times with DMF, MeOH, and DCM, detected by ninhydrin, the resin was colorless and transparent, the solution was light yellow, and the reaction was complete. 25% 4-methylpiperidine/DMF (volume ratio) was added to remove the Fmoc group. Cycled in sequence to complete the coupling of the backbone sequence. Herein, Fmoc-Lys(Dde)-OH was used for K²⁷, and Boc-Gly-OH was used for the N-terminus. Subsequently, 2% hydrazine hydrate/DMF was added to the peptide resin, 10 min * 3 times, to remove the Dde of the Lys side chain, and then FMOC-AEEA-OH, FMOC-AEEA-OH, FMOC-Glu-α-OtBu, and OtBu-ODDA were condensed in sequence. The peptide resin was suction dried, 15 mL of pre-cooled cleavage solution (TFA:TIS:H₂O=95:2.5:2.5) was added, reacted with stirring at room temperature for 5 h, the reaction solution was drawn out, and the resin was washed 2 times with a small amount of TFA and drawn out. The reaction solutions were combined, pre-cooled methyl tert-butyl ether was added to precipitate a white precipitate, centrifuged, the supernatant was poured off, methyl tert-butyl ether was added again to the precipitate, vortexed, centrifuged, and the supernatant was discarded. The precipitate was placed in a vacuum desiccator and dried for 12 h to obtain a crude product. The crude peptide was dissolved in water, sonicated, filtered, and the filtrate was purified by preparative liquid chromatography. Preparative column: C18-10-100, 30*250 mm. Flow rate: 25 mL/min. Phase A: 0.1% TFA/water, Phase B: 0.1% TFA/90% acetonitrile/water. Gradient elution was performed to obtain the pure target compound. The target compound fractions with a purity >95% were combined, the acetonitrile was rotary evaporated under reduced pressure, and freeze-dried to obtain the target compound. Identified by mass spectrometry, the molecular weight was correct ([M+6]⁶⁺=835.1).

### 2. Synthesis of PW-98

The peptide sequence was synthesized with reference to the method for PW-89. Herein, Boc-Gly-OH was used for the N-terminus, Fmoc-Lys(Alloc)-OH was used for K²⁷, and Fmoc-Glu(OAll)-OH was used for E³¹. Afterwards, under nitrogen protection, a DCM solution of 0.3 equivalents of tetrakis(triphenylphosphine)palladium and 12 equivalents of phenylsilane was added to the peptide resin, and reacted at room temperature for 2 h, to remove the allyl ester of the Glu side chain and the allyloxycarbonyl of the Lys side chain. Suction filtered, washed 5 times with DCM and 3 times with DMF, 2 equivalents of PYBOP, 2 equivalents of HOBt, and 4 equivalents of DIEA dissolved in DMF were added, and reacted at room temperature for 5 h. Suction filtered, washed 3 times with DMF and 3 times with DCM, and detected by ninhydrin; if the reaction was incomplete, the condensation was repeated once. Afterwards, with reference to the method for PW-89, cleavage and purification were performed to obtain the pure target compound. Identified by mass spectrometry, the molecular weight was correct ([M+6]⁶⁺=713.0).

### 3. Synthesis of PW-71

With reference to the synthesis method of PW-89, the pure target compound was obtained. Identified by mass spectrometry, the molecular weight was correct ([M+7]⁷⁺=720.1).

### 4. Synthesis of PW-72

With reference to the synthesis method of PW-89, the pure target compound was obtained. Identified by mass spectrometry, the molecular weight was correct ([M+7]⁷⁺=720.1).

### 5. Synthesis of PW-73

With reference to the synthesis method of PW-89, the pure target compound was obtained. Identified by mass spectrometry, the molecular weight was correct ([M+7]⁷⁺=722.0).

### 6. Synthesis of PW-74

With reference to the synthesis method of PW-98, the pure target compound was obtained. Identified by mass spectrometry, the molecular weight was correct ([M+5]⁵⁺=861.2).

### 7. Synthesis of PW-75

With reference to the synthesis method of PW-98, the pure target compound was obtained. Identified by mass spectrometry, the molecular weight was correct ([M+5]⁵⁺=861.2).

### 8. Synthesis of PW-90

With reference to the synthesis method of PW-89, the pure target compound was obtained. Identified by mass spectrometry, the molecular weight was correct ([M+7]⁷⁺=701.3).

### 9. Synthesis of PW-92

With reference to the synthesis method of PW-89, the pure target compound was obtained. Identified by mass spectrometry, the molecular weight was correct ([M+7]⁷⁺=716.0).

### 10. Synthesis of PW-93

With reference to the synthesis method of PW-89, the pure target compound was obtained. Identified by mass spectrometry, the molecular weight was correct ([M+7]⁷⁺=701.2).

### 11. Synthesis of PW-95

With reference to the synthesis method of PW-89, the pure target compound was obtained. Identified by mass spectrometry, the molecular weight was correct ([M+7]⁷⁺=718.0).

### 12. Synthesis of PW-96

With reference to the synthesis method of PW-89, the pure target compound was obtained. Identified by mass spectrometry, the molecular weight was correct ([M+7]⁷⁺=703.1).

### 13. Synthesis of PW-99

With reference to the synthesis method of PW-98, the pure target compound was obtained. Identified by mass spectrometry, the molecular weight was correct ([M+7]⁷⁺=596.4).

### 14. Synthesis of PW-101

With reference to the synthesis method of PW-98, the pure target compound was obtained. Identified by mass spectrometry, the molecular weight was correct ([M+6]⁶⁺=712.7).

### 15. Synthesis of PW-102

With reference to the synthesis method of PW-98, the pure target compound was obtained. Identified by mass spectrometry, the molecular weight was correct ([M+7]⁷⁺=596.4).

### 16. Synthesis of PW-106

With reference to the synthesis method of PW-98, the pure target compound was obtained. Identified by mass spectrometry, the molecular weight was correct ([M+6]⁶⁺=712.7).

### 17. Synthesis of PW-107

With reference to the synthesis method of PW-98, the pure target compound was obtained. Identified by mass spectrometry, the molecular weight was correct ([M+6]⁶⁺=695.8).

### 18. Synthesis of PW-109

With reference to the synthesis method of PW-98, the pure target compound was obtained. Identified by mass spectrometry, the molecular weight was correct ([M+6]⁶⁺=712.7).

### 19. Synthesis of PW-110

With reference to the synthesis method of PW-98, the pure target compound was obtained. Identified by mass spectrometry, the molecular weight was correct ([M+4]⁴⁺=1042.9).

### 20. Synthesis of PW-160

With reference to the synthesis method of PW-98, the pure target compound was obtained. Identified by mass spectrometry, the molecular weight was correct ([M+4]⁴⁺=1042.9).

### 21. Synthesis of PW-161

With reference to the synthesis method of PW-98, the pure target compound was obtained. Identified by mass spectrometry, the molecular weight was correct ([M+6]⁶⁺=712.7).

### 22. Synthesis of PW-164

With reference to the synthesis method of PW-98, first, the synthesis of the C-terminal 9-peptide was completed, wherein Fmoc-Lys(Alloc)-OH was used for K²⁶, and Fmoc-Glu(OAll)-OH was used for E³². Then, with reference to the synthesis method of PW-98, the first cyclization was completed. Then, the synthesis of the remaining sequence was continued and completed, wherein Boc-Gly-OH was used for the N-terminus, Fmoc-Lys(Alloc)-OH was used for K²⁰, and Fmoc-Glu(OAll)-OH was used for E¹⁷. Then, with reference to the synthesis method of PW-98, the second cyclization was completed, and cleavage and purification were performed to obtain the pure target compound. Identified by mass spectrometry, the molecular weight was correct ([M+6]⁶⁺=692.7).

### 23. Synthesis of PW-165

With reference to the synthesis method of PW-164, the pure target compound was obtained. Identified by mass spectrometry, the molecular weight was correct ([M+6]⁶⁺=709.8).

### 24. Synthesis of PW-170

With reference to the synthesis method of PW-98, the pure target compound was obtained. Identified by mass spectrometry, the molecular weight was correct ([M+6]⁶⁺=712.8).

### 25. Synthesis of PW-173

With reference to the synthesis method of PW-98, the synthesis of the backbone was completed, wherein Boc-Gly-OH was used for the N-terminus, Fmoc-Lys(Dde)-OH was used for K²¹, Fmoc-Lys(Alloc)-OH was used for K²⁷, and Fmoc-Glu(OAll)-OH was used for E³¹. Afterwards, with reference to the synthesis method of PW-89, the Dde was removed to complete the side chain synthesis; then, with reference to the synthesis method of PW-98, the cyclization was completed. Finally, cleavage and purification were performed to obtain the pure target compound, and identified by mass spectrometry, the molecular weight was correct ([M+6]⁶⁺=832.1).

### 26. Synthesis of PW-174

With reference to the synthesis method of PW-173, the pure target compound was obtained. Identified by mass spectrometry, the molecular weight was correct ([M+6]⁶⁺=814.8).

### 27. Synthesis of PW-175

With reference to the synthesis method of PW-173, the pure target compound was obtained. Identified by mass spectrometry, the molecular weight was correct ([M+6]⁶⁺=832.1).

### 28. Synthesis of PW-176

With reference to the synthesis method of PW-173, the pure target compound was obtained. Identified by mass spectrometry, the molecular weight was correct ([M+6]⁶⁺=814.8).

### 29. Synthesis of PW-177

With reference to the synthesis method of PW-173, the pure target compound was obtained. Identified by mass spectrometry, the molecular weight was correct ([M+6]⁶⁺=832.1).

### 30. Synthesis of PW-178

With reference to the synthesis method of PW-173, the pure target compound was obtained. Identified by mass spectrometry, the molecular weight was correct ([M+6]⁶⁺=814.8).

### 31. Synthesis of PW-179

With reference to the synthesis method of PW-173, the pure target compound was obtained. Identified by mass spectrometry, the molecular weight was correct ([M+6]⁶⁺=832.1).

### 32. Synthesis of PW-180

With reference to the synthesis method of PW-173, the pure target compound was obtained. Identified by mass spectrometry, the molecular weight was correct ([M+6]⁶⁺=814.8).

### 33. Synthesis of PW-181

With reference to the synthesis method of PW-89, the pure target compound was obtained. Identified by mass spectrometry, the molecular weight was correct ([M+6]⁶⁺=835.1).

### 34. Synthesis of PW-182

With reference to the synthesis method of PW-89, the pure target compound was obtained. Identified by mass spectrometry, the molecular weight was correct ([M+7]⁷⁺=701.2).

### 35. Synthesis of PW-337

With reference to the synthesis method of PW-89, the pure target compound was obtained. Identified by mass spectrometry, the molecular weight was correct ([M+4]⁴⁺=1234.1).

### 36. Synthesis of PW-183

With reference to the synthesis method of PW-89, the pure target compound was obtained. Identified by mass spectrometry, the molecular weight was correct ([M+6]⁶⁺=835.1).

### 37. Synthesis of PW-184

With reference to the synthesis method of PW-89, the pure target compound was obtained. Identified by mass spectrometry, the molecular weight was correct ([M+7]⁷⁺=701.2).

### 38. Synthesis of PW-185

With reference to the synthesis method of PW-89, the pure target compound was obtained. Identified by mass spectrometry, the molecular weight was correct ([M+6]⁶⁺=837.5).

### 39. Synthesis of PW-186

With reference to the synthesis method of PW-89, the pure target compound was obtained. Identified by mass spectrometry, the molecular weight was correct ([M+6]⁶⁺=820.1).

### 40. Synthesis of PW-239

With reference to the synthesis method of PW-98, the pure target compound was obtained. Identified by mass spectrometry, the molecular weight was correct ([M+6]⁶⁺=698.6).

### 41. Synthesis of PW-240

With reference to the synthesis method of PW-98, the pure target compound was obtained. Identified by mass spectrometry, the molecular weight was correct ([M+6]⁶⁺=681.4).

### 42. Synthesis of PW-241

With reference to the synthesis method of PW-89, the pure target compound was obtained. Identified by mass spectrometry, the molecular weight was correct ([M+6]⁶⁺=820.8).

### 43. Synthesis of PW-242

With reference to the synthesis method of PW-89, the pure target compound was obtained. Identified by mass spectrometry, the molecular weight was correct ([M+6]⁶⁺=803.7).

### 44. Synthesis of PW-247

With reference to the synthesis method of PW-89, the pure target compound was obtained. Identified by mass spectrometry, the molecular weight was correct ([M+6]⁶⁺=830.3).

### 45. Synthesis of PW-252

With reference to the synthesis method of PW-89, the pure target compound was obtained. Identified by mass spectrometry, the molecular weight was correct ([M+5]⁵⁺=990.4).

### 46. Synthesis of PW-253

With reference to the synthesis method of PW-89, the pure target compound was obtained. Identified by mass spectrometry, the molecular weight was correct ([M+5]⁵⁺=969.4).

### 47. Synthesis of PW-257

With reference to the synthesis method of PW-89, the pure target compound was obtained. Identified by mass spectrometry, the molecular weight was correct ([M+7]⁷⁺=680.5).

### 48. Synthesis of PW-259

With reference to the synthesis method of PW-89, the pure target compound was obtained. Identified by mass spectrometry, the molecular weight was correct ([M+5]⁵⁺=940.7).

### 49. Synthesis of PW-261

With reference to the synthesis method of PW-89, the pure target compound was obtained. Identified by mass spectrometry, the molecular weight was correct ([M+6]⁶⁺=774.7).

### Experimental Example Biological Activity Assay

### 1. Axonal growth of SH-SY5Y cells

After differentiation induced by retinoic acid (RA), the SH-SY5Y (human neuroblastoma) cell line exhibited a morphology and gene expression similar to those of neurons, and can express PTPσ membrane receptors. Therefore, RA-induced differentiated SH-SY5Y can be used to simulate neuronal axonal growth in vitro and to test the effects of PW polypeptides on axonal growth.

Preparation of culture medium for SH-SY5Y: 1) DMEM/F12 + 10% FBS + 1 × penicillin-streptomycin + 1 mM sodium pyruvate; 2) DMEM/F12 + 1% FBS + 1 × penicillin-streptomycin + 1 mM sodium pyruvate. Preparation of 50 mM retinoic acid stock solution: 15.02 mg of retinoic acid was weighed and dissolved in 1 mL of dimethyl sulfoxide (DMSO), aliquoted, and frozen at -80°C. Proteoglycan (ACAN/CSPG1) is a type of chondroitin sulfate proteoglycan (CSPG). Preparation of 1 mg/mL proteoglycan stock solution: 1 mg of ACAN solid was dissolved in 1 mL of distilled water, aliquoted, and frozen at -80°C. Preparation of PW polypeptide solution for cells (1 mM): the corresponding mass of polypeptide powder was weighed, dissolved in PBS and filtered through a 0.2 µm filter membrane, aliquoted, and frozen at -20°C.

The 1 mg/mL proteoglycan was diluted to 250 µg/mL with distilled water to coat a 24-well cell culture plate: each well was coated with 3 droplets of ACAN, 2 µL per droplet. Using the culture medium containing 10% FBS, SH-SY5Y cells were seeded in the proteoglycan-coated 24-well plate (20,000 cells/well) and incubated (37°C, 5% CO₂) for 20 h. The medium was replaced with a culture medium containing 1% FBS, and PW polypeptide (2.5 µM) or an equal volume of PBS solution was added. After incubation for 72 h, the cells were fixed with 4% paraformaldehyde (room temperature, 30 min), and cell morphology was photographed and recorded using a 10× optical microscope. 20-30 cells were randomly selected under each field of view, and axonal length was measured using Image J-SNT.

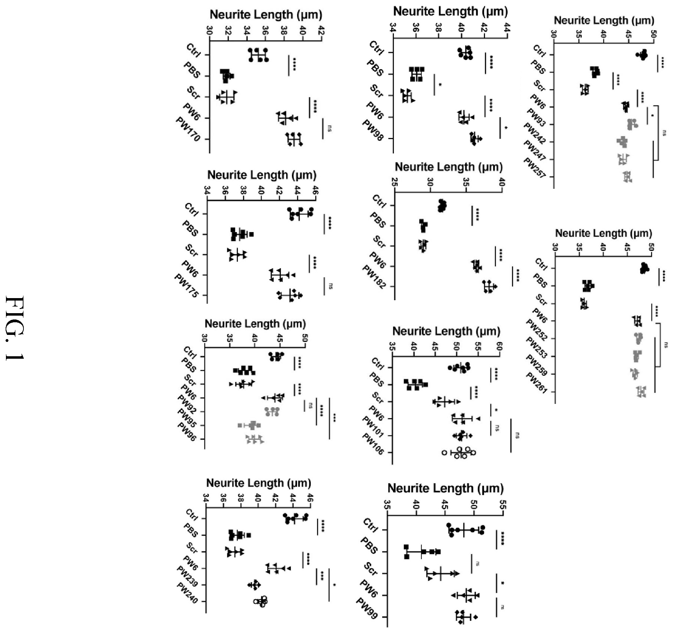

Data analysis: All measured axonal lengths within each proteoglycan-coated range were averaged to serve as one data point. One-way analysis of variance and Tukey's test were used to compare the differences between groups. P<0.05 was considered as a significant difference. The experimental results are shown in FIG. 1.

The results in FIG. 1 show that, compared with the normal cell control (Ctrl), proteoglycan (ACAN) significantly inhibited the axonal growth of SH-SY5Y cells (PBS group). Compared with the scrambled polypeptide (Scr, negative control), PW6 (positive control), PW92, PW93, PW98, PW99, PW101, PW106, PW170, PW175, PW182, PW239, PW240, PW242, PW247, PW252, PW253, PW257, PW259, and PW261 all showed a significant effect of promoting axonal growth. Compared with PW6, the promoting effect of PW93, PW98, and PW182 on axonal growth was significantly enhanced. The promoting effect of PW92, PW99, PW101, PW106, PW170, PW175, PW242, PW247, PW252, PW253, PW257, PW259, and PW261 on axonal growth had no significant difference compared with PW6. The promoting effect of PW95, PW96, PW239, and PW240 on axonal growth was not significantly enhanced compared with PW6. Each group of data is expressed as mean ± standard deviation; *p<0.05, **p<0.01, ***p<0.001, ****p<0.0001; one-way analysis of variance and Tukey's test.

### 2. Effects of PW337 (1, 2, 4 mg/kg) on the body weight and activity status of rats with spinal cord injury

Female SD rats (250-270 g) were randomly grouped after being weighed. After the dorsal T10 segment lamina of the rats was removed, spinal cord contusion was performed on rats in each spinal cord injury (SCI) surgery group, while for rats in the sham operation (sham) group, only the T10 segment lamina was removed without contusion. Rats with a BBB locomotor score > 1 on day 1 after spinal cord injury surgery were excluded, and the remaining rats were randomly grouped and subcutaneously injected daily with: equal volume of physiological saline (vehicle control), PW5 (1 mg/kg), and PW337 (1, 2, 4 mg/kg); rats in the sham operation group were not injected. The body weight of the rats was measured weekly after spinal cord injury surgery, and BBB scoring and open field activity tests were performed.

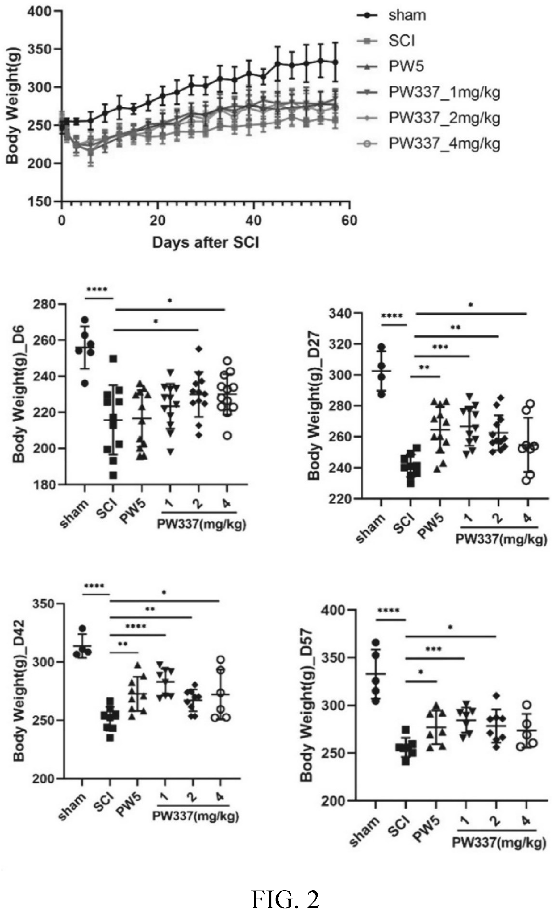

The body weight results in FIG. 2 show that, compared with rats in the spinal cord injury (SCI) group, body weight recovery of rats in the PW337 (2 mg/kg) group significantly accelerated on days 6-57 after surgery; body weight recovery of rats in the PW5 (1 mg/kg) and PW337 (1 mg/kg) groups significantly accelerated on days 27-57 after surgery; body weight recovery of rats in the PW337 (4 mg/kg) group significantly accelerated on days 6-42 after surgery. *p<0.05, **p<0.01, ***p<0.001, ****p<0.0001, t-test.

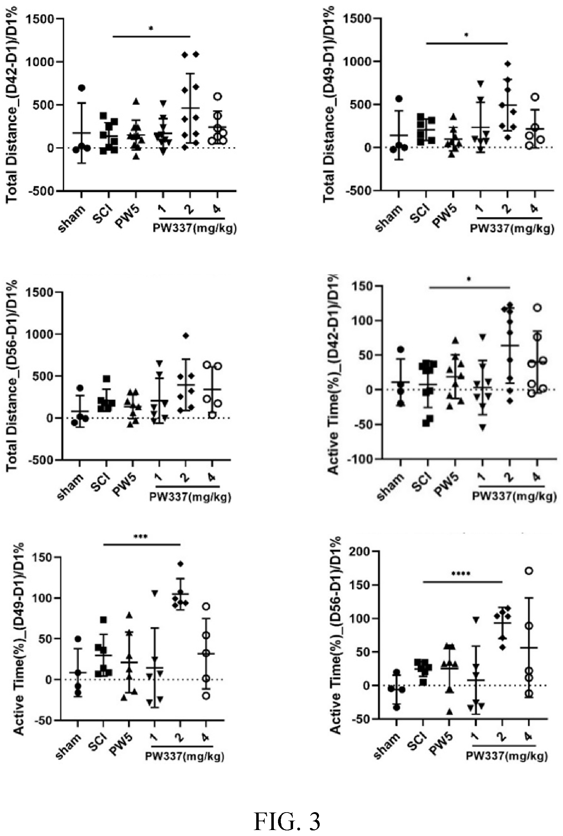

The open field activity results in FIG. 3 show that, at weeks 6 and 7 after surgery compared with day 1 after surgery, the increase (%) in movement distance of rats in the PW337 (2 mg/kg) group was higher than that in the spinal cord injury (SCI) group. At weeks 6-8 after surgery compared with day 1 after surgery, the increase (%) in movement time of rats in the PW337 (2 mg/kg) group was higher than that in the SCI group. *p<0.05, ***p<0.001, ****p<0.0001, t-test.

Combining the experimental results of FIGs. 2-3, it can be found that PW337 can significantly improve the overall status of rats after spinal cord injury surgery, which is manifested as accelerated recovery of body weight and autonomous activity capability. Among them, PW337 at a dose of 2 mg/kg can significantly improve the autonomous activity capability of rats, suggesting that this dose may improve the locomotor function of rats.

### 3. Effects of PW337 (2 mg/kg) on the recovery of locomotor function in rats with chronic spinal cord injury

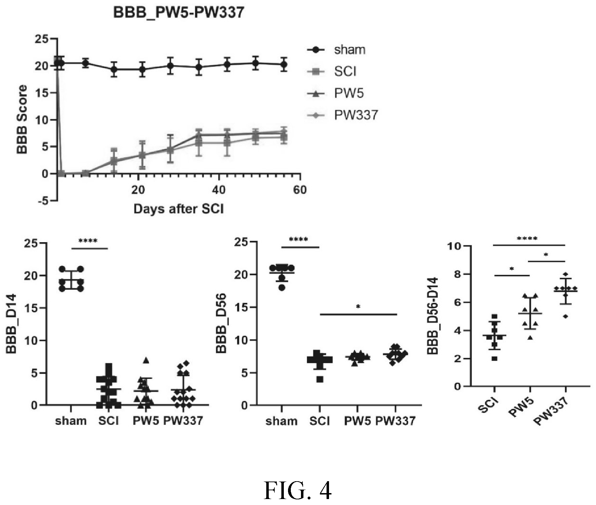

Female SD rats (250-270 g) were randomly grouped after being weighed. After the dorsal T10 segment lamina of the rats was removed, spinal cord contusion was performed on rats in each spinal cord injury (SCI) surgery group, while for rats in the sham operation (sham) group, only the T10 segment lamina was removed without contusion. Rats with a BBB locomotor score > 1 on day 1 after spinal cord injury surgery were excluded, and the remaining rats were randomly grouped and subcutaneously injected daily with: equal volume of physiological saline (vehicle control), PW5 (2 mg/kg), and PW337 (2 mg/kg); rats in the sham operation group were not injected. BBB scoring was performed weekly after spinal cord injury surgery. The BBB scoring results in FIG. 4 show that, on day 1 after surgery, the BBB scores of rats in the spinal cord injury (SCI) group and each dosing group were 0, meeting the inclusion criteria. On days 1-14 after surgery (acute and subacute phase of spinal cord injury), there were no significant differences in BBB scores among rats in the SCI, PW5, and PW337 groups. On day 56 after surgery (chronic phase of spinal cord injury), the BBB score of rats in the PW337 group was significantly higher than that in the SCI group, indicating that PW337 can improve the locomotor function of rats with chronic spinal cord injury. Comparing the BBB scores on day 56 and day 14, it can be found that the increase in BBB score of rats in the PW337 group was significantly higher than that in the PW5 group, indicating that compared with PW5, PW337 can enable faster recovery of locomotor function in rats with chronic spinal cord injury. *p<0.05, ****p<0.0001, t-test.

### 4. Determination of the half-life of polypeptides in rats

The polypeptides in this example were prepared by the preparation examples of the present invention.

Plasma sample collection method: 2 male SD rats (280-300 g) were intravenously injected with PW5, PW6, PW182, and PW337 respectively, the pure peptide dose was 4 mg/kg, and the dosing volume was 2 mL/kg. At 0.5 min, 2 min, 5 min, 10 min, 15 min, 30 min, 60 min, and 120 min after intravenous injection of the drug via the tail vein, blood (400 µL/time) was collected through an indwelling jugular vein catheter into anticoagulant tubes containing EDTA-K2, and 100x protease inhibitor (1:50, v:v; Roche, 11873580001) was added. Whole blood samples were placed on ice and centrifuged (3200 g, 4°C, 10 min) within 1 hour after blood collection. Plasma was taken into a pre-cooled centrifuge tube, protease inhibitor (1:50, v:v) was added again, mixed homogeneously, and stored at -80°C.

Plasma sample processing method: Plasma samples were pipetted into a 96-well plate (50 µL/well), pure methanol (300 µL/well) was added, and mixed by vortexing (1500 rpm, 10 min). Centrifuged (4800 g, 4°C, 10 min), the supernatant was taken into a new 96-well plate (100 µL/well), a 20% aqueous methanol solution containing 0.2% formic acid (100 µL/well) was added, and mixed by vortexing (1500 rpm, 3 min). 10.0 µL was injected for LC-MS/MS analysis to obtain the drug concentration in the samples.
Instrument model: (Waters TQ-XS)
Chromatographic column: SHIMADZU Shim-Pack Scepter Claris C4-300 (2.1×50 mm, 1.9 µm)
Detection results:

**Table 3. Half-life, blood drug concentration, and AUC data of the conjugates**

| Compound No. | Intravenous dosage (mg/kg) | Half-life (min) | 30 min blood drug concentration (ng/mL) | AUClast (0-120 min) |
|---|---|---|---|---|
| PW-5 | 4 | 4.3 | 0 | 928 |
| PW-6 | 4 | 2.2 | 0 | 1787 |
| PW-182 | 4 | 61.6 | 64400 | 4927113 |
| PW-337 | 4 | 95.6 | 56750 | 4157725 |

## Claims

1. A polypeptide derivative or a pharmaceutically acceptable salt thereof, wherein the polypeptide derivative is derived from the following polypeptide sequence:
NH₂-GRKKRRQRRRX₁-DMAEHX₂EX₃LKAX₄DSLKLSX₅EYESI-NH₂,
wherein:
X₁ is selected from C or absent;
X₂ is selected from M or T;
X₃ is selected from R, K, A, F, L, D, E or Cit;
X₄ is selected from N or K;
X₅ is selected from Q or K;
and wherein,
when X₁ is C, the polypeptide derivative introduces a fatty acid side chain at a position of the polypeptide selected from the group consisting of: position 21, position 23 and position 27, and/or
the polypeptide derivative forms an intramolecular cyclic structure by linking side chains of E and K through an amide bond at positions of the polypeptide selected from the group consisting of: position 15 and position 21, position 18 and position 21, position 27 and position 31, position 27 and position 33, and position 30 and position 33, wherein when position 30 and position 33 are linked, X₅ at position 30 is K;
when X₁ is absent, the polypeptide derivative introduces a fatty acid side chain at a position of the polypeptide selected from the group consisting of: position 20, position 22 and position 26, and/or
the polypeptide derivative forms an intramolecular cyclic structure by linking side chains of E and K through an amide bond at positions of the polypeptide selected from the group consisting of: position 14 and position 20, position 17 and position 20, position 26 and position 30, position 26 and position 32, and position 29 and position 32, wherein when position 29 and position 32 are linked, X₅ at position 29 is K.

2. The polypeptide derivative or the pharmaceutically acceptable salt thereof according to claim 1, wherein the fatty acid side chain is: ***-Y1-Y2-Y3-Y4***;
wherein ***Y1*** is absent; or is -NH-(CH₂-CH₂-O)ₘ₁-(CH₂)ₘ₂-CO-, wherein m1 is a positive integer of 2-10, and m2 is 1 or 2; or is *L*/*D*-Glu, the α- or γ-carboxyl group of which is connected to a side chain amino group of a backbone amino acid of the polypeptide, and the amino group of which is connected to the carboxyl group of ***Y2*** or ***Y3*** or ***Y4***;
***Y2*** is absent; or is -NH-(CH₂-CH₂-O)ₘ₃-(CH₂)ₘ₄-CO-, wherein m3 is a positive integer of 2-10, and m4 is 1 or 2;
***Y3*** is absent; or is -NH-(CH₂-CH₂-O)ₘ₅-(CH₂)ₘ₆-CO-, wherein m5 is a positive integer of 2-10, and m6 is 1 or 2; or is *L*/*D*-Glu, the α- or γ-carboxyl group of which is connected to the side chain amino group of the backbone amino acid of the polypeptide or the amino group of ***Y1*** or the amino group of ***Y2***, and the amino group of which is connected to the carboxyl group of ***Y4***; and
***Y4*** is HOOC-(CH₂)ₙ-CO-, CH₃-(CH₂)ₙ-CO-, palmitoyl (-C16) or lauroyl (-C12), wherein n is a positive integer of 10-20.

3. The polypeptide derivative or the pharmaceutically acceptable salt thereof according to claim 1 or 2, wherein the amino acids in the cell-penetrating peptide tat sequence GRKKRRQRRRC of the polypeptide are all L-amino acids, or are all D-amino acids, or are a mixture of D/L-amino acids; and wherein the C at the C-terminus of the cell-penetrating peptide tat is present or absent.

4. The polypeptide derivative or the pharmaceutically acceptable salt thereof according to any one of claims 1 to 3, wherein the polypeptide derivative is selected from any one of the following:
| Number | Sequences |
|---|---|
| PW-71 | NH2-GrKKRrQrRR-C-DMAEHMERLKANDSLK(-AEEA-AEEA-γE-ODDA)LSQEYESI- NH2 |
| PW-72 | NH2-GrKKRrQrRR-C-DMAEHMERLK(-AEEA-AEEA-γE-ODDA)ANDSLKLSQEYESI- NH2 |
| PW-73 | NH2-GrKKRrQrRR-C-DMAEHMERLKAK(-AEEA-AEEA-γE-ODDA)DSLKLSQEYESI- NH2 |
| PW-74 | NH₂-GrKKRrQrRR-C-DMAEHMERLKANDSLcyclo(KLSQE)YESI-NH₂ |
| PW-75 | NH₂-GrKKRrQrRR-C-DMAEHMcyclo(ERLK)ANDSLKLSQEYESI-NH₂ |
| PW-89 | NH2-GrKKRrQrRR-C-DMAEHTERLKANDSLK(-AEEA-AEEA-γE-ODDA)LSQEYESI- NH2 |
| PW-90 | NH₂-GrKKRrQrRR-DMAEHTERLKANDSLK(***-AEEA-AEEA-γE-ODDA***)LSQEYESI-NH₂ |
| PW-92 | NH2-GrKKRrQrRR-C-DMAEHTERLK(-AEEA-AEEA-γE-ODDA)ANDSLKLSQEYESI- NH2 |
| PW-93 | NH₂-GrKKRrQrRR-DMAEHTERLK(***-AEEA-AEEA-γE-ODDA***)ANDSLKLSQEYESI-NH₂ |
| PW-95 | NH2-GrKKRrQrRR-C-DMAEHTERLKAK(-AEEA-AEEA-γE-ODDA)DSLKLSQEYESI- NH2 |
| PW-96 | NH₂-GrKKRrQrRR-DMAEHTERLKAK(***-AEEA-AEEA-γE-ODDA***)DSLKLSQEYESI-NH₂ |
| PW-98 | NH₂-GrKKRrQrRR-C-DMAEHTERLKANDSLcyclo(KLSQE)YESI-NH₂ |
| PW-99 | NH₂-GrKKRrQrRR-DMAEHTERLKANDSLcyclo(KLSQE)YESI-NH₂ |
| PW-101 | NH₂-GrKKRrQrRR-C-DMAEHTcyclo(ERLK)ANDSLKLSQEYESI-NH₂ |
| PW-102 | NH₂-GrKKRrQrRR-DMAEHTcyclo(ERLK)ANDSLKLSQEYESI-NH₂ |
| PW-106 | NH₂-GrKKRrQrRR-C-DMAEHTERLKANDSLcyclo(KLSQEYE)SI-NH₂ |
| PW-107 | NH₂-GrKKRrQrRR-DMAEHTERLKANDSLcyclo(KLSQEYE)SI-NH₂ |
| PW-109 | NH₂-GrKKRrQrRR-C-DMAcyclo(EHTERLK)ANDSLKLSQEYESI-NH₂ |
| PW-110 | NH₂-GrKKRrQrRR-DMAcyclo(EHTERLK)ANDSLKLSQEYESI-NH₂ |
| PW-160 | NH₂-GrKKRrQrRR-DMAEHTERLKANDSLKLScyclo(KEYE)SI-NH₂ |
| PW-161 | NH₂-GrKKRrQrRR-C-DMAEHTERLKANDSLKLScyclo(KEYE)SI-NH₂ |
| PW-164 | NH₂-GrKKRrQrRR-DMAEHTcyclo(ERLK)ANDSLcyclo(KLSQEYE)SI-NH₂ |
| PW-165 | NH₂-GrKKRrQrRR-C-DMAEHTcyclo(ERLK)ANDSLcyclo(KLSQEYE)SI-NH₂ |
| PW-170 | NH₂-GRKKRRQRRR-C-DMAEHTERLKANDSLcyclo(KLSQE)YESI-NH₂ |
| PW-173 | NH2-GrKKRrQrRR-C-DMAEHTERLK(-AEEA-AEEA-γE- ODDA)ANDSLcyclo(KLSQE)YESI-NH2 |
| PW-174 | NH2-GrKKRrQrRR-DMAEHTERLK(-AEEA-AEEA-γE- ODDA)ANDSLcyclo(KLSQE)YESI-NH2 |
| PW-175 | NH2-GRKKRRQRRR-C-DMAEHTERLK(-AEEA-AEEA-γE- ODDA)ANDSLcyclo(KLSQE)YESI-NH2 |
| PW-176 | NH2-GRKKRRQRRR-DMAEHTERLK(-AEEA-AEEA-γE- ODDA)ANDSLcyclo(KLSQE)YESI-NH2 |
| PW-177 | NH2-GrKKRrQrRR-C-DMAEHTcyclo(ERLK)ANDSLK(-AEEA-AEEA-γE- ODDA)LSQEYESI-NH2 |
| PW-178 | NH2-GrKKRrQrRR-DMAEHTcyclo(ERLK)ANDSLK(-AEEA-AEEA-γE- ODDA)LSQEYESI-NH2 |
| PW-179 | NH2-GRKKRRQRRR-C-DMAEHTcyclo(ERLK)ANDSLK(-AEEA-AEEA-γE- ODDA)LSQEYESI-NH2 |
| PW-180 | NH2-GRKKRRQRRR-DMAEHTcyclo(ERLK)ANDSLK(-AEEA-AEEA-γE- ODDA)LSQEYESI-NH2 |
| PW-181 | NH2-GRKKRRQRRR-C-DMAEHTERLKANDSLK(-AEEA-AEEA-γE-ODDA)LSQEYESI- NH2 |
| PW-182 | NH2-GRKKRRQRRR-DMAEHTERLKANDSLK(-AEEA-AEEA-γE-ODDA)LSQEYESI- NH2 |
| PW-337 | NH2-GRKKRRQRRR-DMAEHMERLKANDSLK(-AEEA-AEEA-γE-ODDA)LSQEYESI- NH2 |
| PW-183 | NH2-GRKKRRQRRR-C-DMAEHTERLK(-AEEA-AEEA-γE-ODDA)ANDSLKLSQEYESI- NH2 |
| PW-184 | NH2-GRKKRRQRRR-DMAEHTERLK(-AEEA-AEEA-γE-ODDA)ANDSLKLSQEYESI- NH2 |
| PW-185 | NH2-GRKKRRQRRR-C-DMAEHTERLKAK(-AEEA-AEEA-γE-ODDA)DSLKLSQEYESI- NH2 |
| PW-186 | NH2-GRKKRRQRRR-DMAEHTERLKAK(-AEEA-AEEA-γE-ODDA)DSLKLSQEYESI- NH2 |
| PW-239 | NH₂-GrKKRrQrRR-C-DMAEHTEALKANDSLcyclo(KLSQEYE)SI- NH₂ |
| PW-240 | NH₂-GrKKRrQrRR-DMAEHTEALKANDSLcyclo(KLSQEYE)SI- NH₂ |
| PW-241 | NH2-GrKKRrQrRR-C-DMAEHTEALK(-AEEA-AEEA-γE-ODDA)ANDSLKLSQEYESI- NH2 |
| PW-242 | NH₂-GrKKRrQrRR-DMAEHTEALK(-***AEEA-AEEA-γE-ODDA***)ANDSLKLSQEYESI-NH₂ |
| PW-247 | NH2-GRKKRRQRRR-C-DMAEHTEK(-AEEA-AEEA-γE-ODDA)LKANDSLKLSQEYESI- NH2 |
| PW-252 | NH₂-GrKKRrQrRR-C-DMAEHTERLK(-***AEEA-AEEA-γE-C16***)ANDSLKLSQEYESI-NH₂ |
| PW-253 | NH₂-GrKKRrQrRR-DMAEHTERLK(-***AEEA-AEEA-γE-C16***)ANDSLKLSQEYESI-NH₂ |
| PW-257 | NH₂-GrKKRrQrRR-DMAEHTERLK(-***AEEA-γE-ODDA***)ANDSLKLSQEYESI-NH₂ |
| PW-259 | NH₂-GrKKRrQrRR-DMAEHTERLK(-***AEEA-γE-C16***)ANDSLKLSQEYESI-NH₂ |
| PW-261 | NH₂-GrKKRrQrRR-DMAEHTERLK(-***AEEA-γE-C1***2)ANDSLKLSQEYESI-NH₂ . |

5. A pharmaceutical composition comprising the polypeptide derivative or the pharmaceutically acceptable salt thereof according to any one of claims 1-4.

6. The pharmaceutical composition according to claim 5, wherein the pharmaceutical composition further comprises one or more pharmaceutically acceptable components selected from the following: diluents, buffers, excipients, salts, emulsifiers, osmotic pressure regulating agents, preservatives, stabilizers, and fillers.

7. The pharmaceutical composition according to claim 5, wherein the pharmaceutical composition is administered orally, intravenously, intradermally, transdermally, intrathecally, intraarterially, intraperitoneally, intranasally, intravaginally, intrarectally, intravesically, intratumorally, topically, intramuscularly, subcutaneously, mucosally, by inhalation, by injection, by infusion, or any combination thereof.

8. Use of the polypeptide derivative or the pharmaceutically acceptable salt thereof according to any one of claims 1-4 or the pharmaceutical composition according to any one of claims 5-7 in the preparation of a medicament for treating a disease associated with nerve injury.

9. The use according to claim 8, wherein the disease associated with nerve injury is selected from one or more of spinal cord injury, peripheral nerve injury, traumatic brain injury, stroke, multiple sclerosis, Alzheimer's disease, amyotrophic lateral sclerosis, dementia, and Parkinson's disease.

10. A method for treating a disease associated with nerve injury, the method comprising administering to a subject in need thereof the polypeptide derivative or the pharmaceutically acceptable salt thereof according to any one of claims 1-4 or the pharmaceutical composition according to any one of claims 5-7.

11. The method according to claim 10, wherein the disease associated with nerve injury is selected from one or more of spinal cord injury, peripheral nerve injury, traumatic brain injury, stroke, multiple sclerosis, Alzheimer's disease, amyotrophic lateral sclerosis, dementia, and Parkinson's disease.

12. The polypeptide derivative or the pharmaceutically acceptable salt thereof according to any one of claims 1-4 or the pharmaceutical composition according to any one of claims 5-7 for use in treating a disease associated with nerve injury.

13. The polypeptide derivative or the pharmaceutically acceptable salt thereof or the pharmaceutical composition for use according to claim 12, wherein the disease associated with nerve injury is selected from one or more of spinal cord injury, peripheral nerve injury, traumatic brain injury, stroke, multiple sclerosis, Alzheimer's disease, amyotrophic lateral sclerosis, dementia, and Parkinson's disease.
